# EUROPEAN PATENT SPECIFICATION

(11) **EP 2 517 010 B1**
(45) Date of publication and mention of the grant of the patent: **05.06.2019**
(21) Application number: 10843594.2
(22) Date of filing: 22.12.2010
(51) Int. Cl.: G01N 33/50, G01N 33/68

(54) **EPIDERMAL GROWTH FACTOR RECEPTOR (EGFR) PROTEIN SRM/MRM ASSAY**
SRM/MRM-TEST AUF DAS REZEPTORPROTEIN DES EPIDERMALEN WACHSTUMSFAKTORS (EGFR)
DOSAGE SRM/MRM DE LA PROTÉINE DU RÉCEPTEUR DU FACTEUR DE CROISSANCE DE L'ÉPIDERME (EGFR)

(30) Priority: 22.12.2009 US 289384 P
(43) Date of publication of application: 31.10.2012
(73) Proprietor: Expression Pathology, Inc., Rockville, MD 20850 (US)
(72) Inventor: KRIZMAN, David, B., Gaithersburg, MD 20882 (US); HEMBROUGH, Todd, Gaithersburg, MD 20882 (US); THYPARAMBIL, Sheeno, Frederick,MD 21704 (US)
(74) Representative: Bohmann, Armin K.
(86) International application number: PCT/US2010/061916
(87) International publication number: WO 2011/087865

(56) References cited:
- WO-A2-2009/074350
- US-A1- 2008 108 795
- TAYLOR P ET AL: "PP118 Detection and quantification of EGF receptor phosphorylation in formalin-fixed tumor sections by selected/multiple reaction monitoring mass spectrometry", EUROPEAN JOURNAL OF CANCER. SUPPLEMENT, PERGAMON, OXFORD, GB, vol. 7, no. 4, 1 October 2009 (2009-10-01) , page 31, XP026692231, ISSN: 1359-6349, DOI: 10.1016/S1359-6349(09)72213-X [retrieved on 2009-10-01]
- YAN LI ET AL: "Highly selective and rapid enrichment of phosphorylated peptides using gallium oxide-coated magnetic microspheres for MALDI-TOF-MS and nano-LC-ESI-MS/MS/MS analysis", PROTEOMICS, vol. 8, no. 2, 1 January 2008 (2008-01-01) , pages 238-249, XP055065869, ISSN: 1615-9853, DOI: 10.1002/pmic.200700454
- BECKER K-F ET AL: "Quantitative protein analysis from formalin-fixed tissues: implications for translational clinical research and nanoscale molecular diagnosis", JOURNAL OF PATHOLOGY, JOHN WILEY & SONS LTD, GB, vol. 211, no. 3, 1 February 2007 (2007-02-01), pages 370-378, XP002515221, ISSN: 0022-3417, DOI: 10.1002/PATH.2107
- HOOD B L ET AL: "Proteomic analysis of formalin-fixed prostate cancer tissue", MOLECULAR & CELLULAR PROTEOMICS, AMERICAN SOCIETY FOR BIOCHEMISTRY AND MOLECULAR BIOLOGY, INC, US, vol. 4, no. 11, 1 November 2005 (2005-11-01), pages 1741-1753, XP002482477, ISSN: 1535-9476, DOI: 10.1074/MCP.M500102-MCP200 [retrieved on 2005-08-09]
- PRIETO ET AL.: 'Liquid Tissue: proteomic profiling of formalin-fixed tissues.' BIO TECHNIQUES. vol. 38, June 2005, pages S32 - S35
- ERBA ET AL.: 'Systematic analysis of the epidermal growth factor receptor by mass spectrometry reveals stimulation-dependent multisite phosphorylation.' MOL CELL PROTEOMICS vol. 4, no. 8, August 2005, pages 1107 - 1121

## Description

### Field

The present invention is related to a method for measuring the level of the human Epidermal Growth Factor Receptor (EGFR) protein in a human biological sample of formalin fixed and paraffin embedded tissue.

### Introduction

Specific peptides derived from subsequences of the Epidermal Growth Factor Receptor protein and which will be referred to as EGFR, and which can also be referred to as the receptor tyrosine-protein kinase ErbB-1 protein, are provided. The peptide sequence and fragmentation/transition ions for each peptide are particularly useful in a mass spectrometry-based Selected Reaction Monitoring (SRM), which can also be referred to as a Multiple Reaction Monitoring (MRM) assay, and will be referred to as SRM/MRM. The use of one such peptide for SRM/MRM quantitative analysis of the EGFR protein is described.

This SRM/MRM assay can be used to measure *relative* or *absolute* quantitative levels of one or more of the specific peptides from the EGFR protein and therefore provide a means of measuring the amount of the EGFR protein in a given protein preparation obtained from a biological sample by mass spectrometry.

More specifically, the SRM/MRM assay can measure these peptides directly in complex protein lysate samples prepared from cells procured from patient tissue samples, such as formalin fixed cancer patient tissue. Methods of preparing protein samples from formalin-fixed tissue are described in U.S. Patent No. 7,473,532. The methods described in U.S. Patent No. 7,473,532 may conveniently be carried out using Liquid Tissue™ reagents and protocol available from Expression Pathology Inc. (Rockville, MD).

The most widely and advantageously available form of tissues from cancer patients tissue is formalin fixed, paraffin embedded tissue. Formaldehyde/formalin fixation of surgically removed tissue is by far and away the most common method of preserving cancer tissue samples worldwide and is the accepted convention for standard pathology practice. Aqueous solutions of formaldehyde are referred to as formalin. "100%" formalin consists of a saturated solution of formaldehyde (this is about 40% by volume or 37% by mass) in water, with a small amount of stabilizer, usually methanol to limit oxidation and degree of polymerization. The most common way in which tissue is preserved is to soak whole tissue for extended periods of time (8 hours to 48 hours) in aqueous formaldehyde, commonly termed 10% neutral buffered formalin, followed by embedding the fixed whole tissue in paraffin wax for long term storage at room temperature. Thus molecular analytical methods to analyze formalin fixed cancer tissue will be the most accepted and heavily utilized methods for analysis of cancer patient tissue.

Results from the SRM/MRM assay can be used to correlate accurate and precise quantitative levels of the EGFR protein within the specific tissue samples (e.g., cancer tissue sample) of the patient or subject from whom the tissue (biological sample) was collected and preserved. This not only provides diagnostic information about the cancer, but also permits a physician or other medical professional to determine appropriate therapy for the patient. Such an assay that provides diagnostically and therapeutically important information about levels of protein expression in a diseased tissue or other patient sample is termed a *companion diagnostic* assay. For example, such an assay can be designed to diagnose the stage or degree of a cancer and determine a therapeutic agent to which a patient is most likely to respond.

Erba EB et al. (Mol Cell Proteomics, vol. no. 4, no. 8, August 2005, pp. 1107-1121) report on a systematic analysis of epidermal growth factor receptor by mass spectrometry revealing stimulation-dependent multisite phosphorylation.

US 2008/0108795 A1 is related to 168 phosphorylation sites identified in signal transduction proteins and pathways down-stream of, and including, EGFR kinase and phosphorylation-site specific antibodies and heavy-isotope labeled peptides for the selective detection and quantification of these phosphorylated sites/proteins, as well as methods of using the reagents for such purpose.

WO 2009/074350 A2 is related to biomarkers for determining EGFR kinase activity in a subject.

Taylor T et al. (European Journal of Cancer. Supplement, vol. 7, no. 4, 1 October 2009, p.31) report on the detection and quantification of EGF receptor phosphorylation in formalin-fixed tumor sections by selected/multiple reaction monitoring mass spectrometry.

### Summary

The problem underlying the present invention is solved by the subject matter of the attached dependent claim; preferred embodiments may be taken from the attached dependent claims.

More specifically, the problem underlying the present invention is solved by a method for measuring the level of the human Epidermal Growth Factor Receptor (EGFR) protein in a human biological sample of formalin fixed and paraffin embedded tissue, wherein the tissue is human breast cancer tissue, the method comprising detecting and quantifying the amount of a EGFR fragment peptide in a protein digest prepared from said human biological sample using mass spectrometry; and calculating the level of EGFR protein in said sample;
wherein the EGFR fragment peptide is the peptide of SEQ ID NO: 4, and
wherein said level is a relative level or an absolute level.

In an embodiment of the method, the method further comprises the step of fractionating said protein digest prior to detecting and quantifying the amount of said EGFR fragment peptide.

In an embodiment of the method, wherein said protein digest comprises a protease digest.

In an embodiment of the method, quantifying said EGFR fragment peptide comprises comparing the amount of said EGFR fragment peptide in one biological sample to the amount of the same EGFR fragment peptide in a different and separate biological sample.

In an embodiment of the method, quantifying said EGFR fragment peptide comprises determining the amount of said EGFR fragment peptide in a biological sample by comparison to an added internal standard peptide of known amount, wherein said EGFR fragment peptide in the biological sample is compared to an internal standard peptide having the same amino acid sequence; and wherein the internal standard peptide is an isotopically labeled peptide.

In an embodiment of the method, detecting and quantifying the amount of said EGFR fragment peptide in the protein digest indicates the presence of modified or unmodified EGFR protein and an association with cancer in the subject.

In an embodiment of the method, the method further comprises correlating the results of said detecting and quantifying the amount of said EGFR fragment peptide, or the level of said EGFR protein to the diagnostic stage/grade/status of the cancer.

In an embodiment of the method, correlating the results of said detecting and quantifying the amount of said EGFR fragment peptide, or the level of said EGFR protein to the diagnostic stage/grade/status of the cancer is combined with detecting and/or quantifying the amount of other proteins or peptides from other proteins in a multiplex format to provide additional information about the diagnostic stage/grade/status of the cancer.

The assays described herein measure *relative* or *absolute* levels of specific unmodified peptides from the EGFR protein and also can measure absolute or relative levels of specific modified peptides from the EGFR protein. Examples of modifications include phosphorylated amino acid residues and glycosylated amino acid residues that are present on the peptides.

*Relative* quantitative levels of the EGFR protein are determined by the SRM/MRM methodology for example by comparing SRM/MRM signature peak areas (e.g., signature peak area or integrated fragment ion intensity) of an individual EGFR peptide in different samples. Alternatively, it is possible to compare multiple SRM/MRM signature peak areas for multiple EGFR signature peptides, where each peptide has its own specific SRM/MRM signature peak, to determine the relative EGFR protein content in one biological sample with the EGFR protein content in one or more additional or different biological samples. In this way, the amount of a particular peptide, or peptides, from the EGFR protein, and therefore the amount of the EGFR protein, is determined relative to the same EGFR peptide, or peptides, across 2 or more biological samples under the same experimental conditions. In addition, relative quantitation can be determined for a given peptide, or peptides, from the EGFR protein within a single sample by comparing the signature peak area for that peptide by SRM/MRM methodology to the signature peak area for another and different peptide, or peptides, from a different protein, or proteins, within the same protein preparation from the biological sample. In this way, the amount of a particular peptide from the EGFR protein, and therefore the amount of the EGFR protein, is determined relative one to another within the same sample. These approaches generate quantitation of an individual peptide, or peptides, from the EGFR protein to the amount of another peptide, or peptides, between samples and within samples wherein the amounts as determined by signature peak area are relative one to another, regardless of the absolute weight to volume or weight to weight amounts of the EGFR peptide in the protein preparation from the biological sample. Relative quantitative data about individual signature peak areas between different samples are normalized to the amount of protein analyzed per sample. Relative quantitation can be performed across many peptides from multiple proteins and the EGFR protein simultaneously in a single sample and/or across many samples to gain insight into relative protein amounts, one peptide/protein with respect to other peptides/proteins.

*Absolute* quantitative levels of the EGFR protein are determined by, for example, the SRM/MRM methodology whereby the SRM/MRM signature peak area of an individual peptide from the EGFR protein in one biological sample is compared to the SRM/MRM signature peak area of a spiked internal standard. In one embodiment, the internal standard is a synthetic version of the same exact EGFR peptide that contains one or more amino acid residues labeled with one or more heavy isotopes. Such isotope labeled internal standards are synthesized so that when analyzed by mass spectrometry it generates a predictable and consistent SRM/MRM signature peak that is different and distinct from the native EGFR peptide signature peak and which can be used as a comparator peak. Thus when the internal standard is spiked into a protein preparation from a biological sample in known amounts and analyzed by mass spectrometry, the SRM/MRM signature peak area of the native peptide is compared to the SRM/MRM signature peak area of the internal standard peptide, and this numerical comparison indicates either the absolute molarity and/or absolute weight of the native peptide present in the original protein preparation from the biological sample. Absolute quantitative data for fragment peptides are displayed according to the amount of protein analyzed per sample. Absolute quantitation can be performed across many peptides, and thus proteins, simultaneously in a single sample and/or across many samples to gain insight into absolute protein amounts in individual biological samples and in entire cohorts of individual samples.

The SRM/MRM assay method can be used to aid diagnosis of the stage of cancer, for example, directly in patient-derived tissue, such as formalin fixed tissue, and to aid in determining which therapeutic agent would be most advantageous for use in treating that patient. Cancer tissue that is removed from a patient either through surgery, such as for therapeutic removal of partial or entire tumors, or through biopsy procedures conducted to determine the presence or absence of suspected disease, is analyzed to determine whether or not a specific protein, or proteins, and which forms of proteins, are present in that patient tissue. Moreover, the expression level of a protein, or multiple proteins, can be determined and compared to a "normal" or reference level found in healthy tissue. Normal or reference levels of proteins found in healthy tissue may be derived from, for example, the relevant tissues of one or more individuals that do not have cancer. Alternatively, normal or reference levels may be obtained for individuals with cancer by analysis of relevant tissues not affected by the cancer. Assays of protein levels (e.g., EGFR levels) can also be used to diagnose the stage of cancer in a patient or subject diagnosed with cancer by employing the EGFR levels. Levels or amounts of proteins or peptides can be defined as the quantity expressed in moles, mass or weight of a protein or peptide determined by the SRM/MRM assay. The level or amount may be normalized to total the level or amount of protein or another component in the lysate analyzed (e.g., expressed in micromoles/microgram of protein or micrograms /microgram of protein). In addition, the level or amount of a protein or peptide may be determined on volume basis, expressed, for example, in micromolar or nanograms/microliter. The level or amount of protein or peptide as determined by the SRM/MRM assay can also be normalized to the number of cells analyzed. Information regarding EGFR can thus be used to aid in determining stage or grade of a cancer by correlating the level of the EGFR protein (or fragment peptides of the EGFR protein) with levels observed in normal tissues. Once the stage and/or grade, and/or EGFR protein expression characteristics of the cancer has been determined, that information can be matched to a list of therapeutic agents (chemical and biological) developed to specifically treat cancer tissue that is characterized by, for example, abnormal expression of the protein or protein(s) (e.g., EGFR) that were assayed. Matching information from an EGFR protein assay to a list of therapeutic agents that specifically targets, for example, the EGFR protein or cells/tissue expressing the protein, defines what has been termed a *personalized medicine* approach to treating disease. The assay methods described herein form the foundation of a *personalized medicine* approach by using analysis of proteins from the patient's own tissue as a source for diagnostic and treatment decisions.

### Brief Description of the Drawings

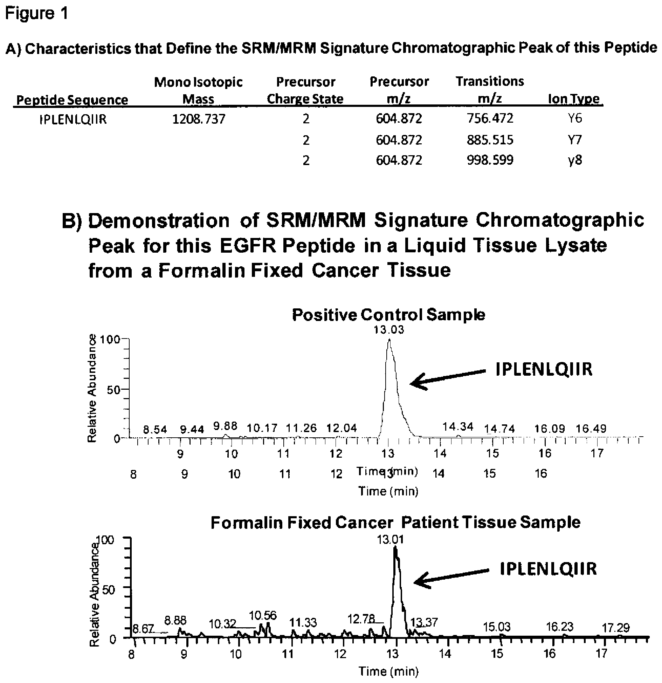

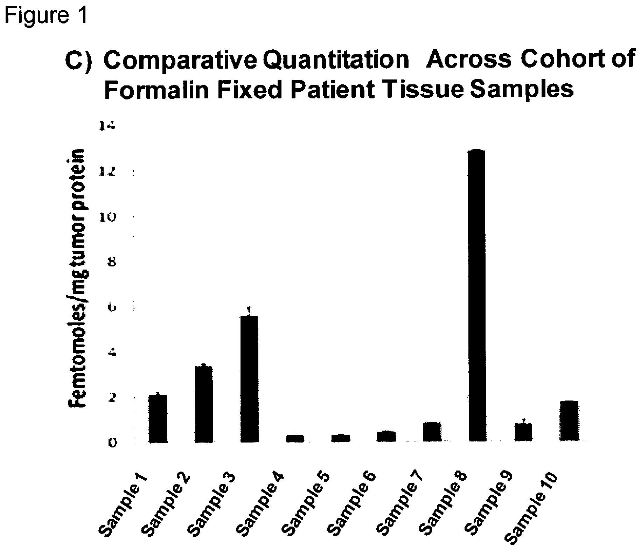

Figure 1, parts A to C, show an example of SRM/MRM assay of a single peptide from the EGFR protein performed on Liquid Tissue™ lysates with quantitation of the EGFR peptide conducted on a triplequadrupole mass spectrometer.

### Detailed Description

In principle, any predicted peptide derived from EGFR protein, prepared for example by digesting with a protease of known specificity (*e.g*. trypsin), can be used as a surrogate reporter to determine the abundance of EGFR protein in a sample using a mass spectrometry-based SRM/MRM assay. Similarly, any predicted peptide sequence containing an amino acid residue at a site that is known to be potentially modified in EGFR protein also might potentially be used to assay the extent of modification of EGFR protein in a sample.

EGFR fragment peptides may be generated by a variety of means including by the use of the Liquid Tissue™ protocol provided in US Patent 7,473,532. The Liquid Tissue™ protocol and reagents are capable of producing peptide samples suitable for mass spectroscopic analysis from formalin fixed paraffin embedded tissue by proteolytic digestion of the proteins in the tissue/biological sample. In the Liquid Tissue™ protocol the tissue/biological is heated in a buffer for an extended period of time (e.g., from about 80° C to about 100° C for a period of time from about 10 minutes to about 4 hours) to reverse or release protein cross-linking. The buffer employed is a neutral buffer, (e.g., a Tris-based buffer, or a buffer containing a detergent). Following heat treatment the tissue/biological sample is treated with one or more proteases, including but not limited to trypsin, chymotrypsin, pepsin, and endoproteinase Lys-C for a time sufficient to disrupt the tissue and cellular structure of said biological sample and to liquefy said sample (e.g., a period of time from 30 minutes to 24 hours at a temperature from 37° C to 65° C). The result of the heating and proteolysis is a liquid, soluble, dilutable biomolecule lysate.

Surprisingly, it was found that many potential peptide sequences from the EGFR protein are unsuitable or ineffective for use in mass spectrometry-based SRM/MRM assays for reasons that are not immediately evident. As it was not possible to predict the most suitable peptides for MRM/SRM assay, it was necessary to experimentally identify modified and unmodified peptides in actual Liquid Tissue™ lysates to develop a reliable and accurate SRM/MRM assay for the EGFR protein. While not wishing to be bound by any theory, it is believed that some peptides might, for example, be difficult to detect by mass spectrometry as they do not ionize well or produce fragments distinct from other proteins, peptides may also fail to resolve well in separation (e.g., liquid chromatography), or adhere to glass or plastic ware.

EGFR peptides found in various embodiments of this disclosure (e.g., Tables 1 and 2) were derived from the EGFR protein by protease digestion of all the proteins within a complex Liquid Tissue™ lysate prepared from cells procured from formalin fixed cancer tissue. Unless noted otherwise, in each instance the protease was trypsin. The Liquid Tissue™ lysate was then analyzed by mass spectrometry to determine those peptides derived from the EGFR protein that are detected and analyzed by mass spectrometry. Identification of a specific preferred subset of peptides for mass-spectrometric analysis is based on; 1) experimental determination of which peptide or peptides from a protein ionize in mass spectrometry analyses of Liquid Tissue™ lysates, and 2) the ability of the peptide to survive the protocol and experimental conditions used in preparing a Liquid Tissue™ lysate. This latter property extends not only to the amino acid sequence of the peptide but also to the ability of a modified amino acid residue within a peptide to survive in modified form during the sample preparation.

**Table 1**

| **Table 1 SEQ ID** | **Peptide Sequence** |
|---|---|
| SEQ ID NO: 1 | CDPSCPNGSCWGAGEENCQKLTKIICAQQCSGR |
| SEQ ID NO: 2 | CEGPCRK |
| SEQ ID NO: 3 | EDSFLQR |
| SEQ ID NO: 4 | IPLENLQIIR |
| SEQ ID NO: 5 | EISDGDVIISGNK |
| SEQ ID NO: 6 | EITGFLLIQAWPENR |
| SEQ ID NO: 7 | ELREATSPKANK |
| SEQ ID NO: 8 | EYHAEGGK |
| SEQ ID NO: 9 | FRELIIEFSK |
| SEQ ID NO: 10 | GLWIPEGEK |
| SEQ ID NO: 11 | GLWIPEGEKVKIPVAIK |
| SEQ ID NO: 12 | HFKNCTSISGDLHILPVAFRGDSFTHTPPLDPQELDILK |
| SEQ ID NO: 13 | IICAQQCSGRCRGK |
| SEQ ID NO: 14 | IPSIATGMVGALLLLLVVALGIGLFMRRR |
| SEQ ID NO: 15 | CEGPCR |
| SEQ ID NO: 16 | LFGTSGQKTK |
| SEQ ID NO: 17 | LTKIICAQQCSGR |
| SEQ ID NO: 18 | NCTSISGDLHILPVAFRGDSFTHTPPLDPQELDILK |
| SEQ ID NO: 19 | PYDGIPASEISSILEKGER |
| SEQ ID NO: 20 | PAGSVQNPVYHNQPLNPAPSR |
| SEQ ID NO: 21 | SPSDCCHNQCAAGCTGPRESDCLVCR |
| SEQ ID NO: 22 | ITDFGLAK |
| SEQ ID NO: 23 | TPQHVKITDFGLAKLLGAEEK |
| SEQ ID NO: 24 | VCNGIGIGEFK |
| SEQ ID NO: 25 | VCNGIGIGEFKDSLSINATNIKHFK |
| SEQ ID NO: 26 | VLGSGAFGTVYKGLWIPEGEKVK |
| SEQ ID NO: 27 | YSFGATCVKKCPR |
| SEQ ID NO: 28 | CRGKSPSDCCHNQCAAGCTGPR |
| SEQ ID NO: 29 | DIVSSDFLSNMSMDFQNHLGSCQK |
| SEQ ID NO: 30 | EFVENSECIQCHPECLPQAMNITCTGR |
| SEQ ID NO: 31 | ELIIEFSKMARDPQR |
| SEQ ID NO: 32 | ELVEPLTPSGEAPNQALLR |
| SEQ ID NO: 33 | ESDCLVCRKFR |
| SEQ ID NO: 34 | GDSFTHTPPLDPQELDILK |
| SEQ ID NO: 35 | GENSCKATGQVCHALCSPEGCWGPEPR |
| SEQ ID NO: 36 | GKSPSDCCHNQCAAGCTGPRESDCLVCR |
| SEQ ID NO: 37 | GRECVDKCNLLEGEPR |
| SEQ ID NO: 38 | ILKETEFKK |
| SEQ ID NO: 39 | IPLENLQIIR |
| SEQ ID NO: 40 | KVCNGIGIGEFK |
| SEQ ID NO: 41 | KVCNGIGIGEFKDSLSINATNIK |
| SEQ ID NO: 42 | LLQERELVEPLTPSGEAPNQALLR |
| SEQ ID NO: 43 | MHLPSPTDSNFYR |
| SEQ ID NO: 44 | NVLVKTPQHVKITDFGLAK |
| SEQ ID NO: 45 | NVSRGRECVDK |
| SEQ ID NO: 46 | NYDLSFLK |
| SEQ ID NO: 47 | PKFRELIIEFSK |
| SEQ ID NO: 48 | LLQERELVEPLTPSGEAPNQALLR |
| SEQ ID NO: 49 | SLKEISDGDVIISGNK |
| SEQ ID NO: 50 | TDLHAFENLEIIR |
| SEQ ID NO: 51 | TDLHAFENLEIIRGR |
| SEQ ID NO: 52 | TKQHGQFSLAVVSLNITSLGLR |
| SEQ ID NO: 53 | TLRRLLQER |
| SEQ ID NO: 54 | TPLLSSLSATSNNSTVACIDR |
| SEQ ID NO: 55 | VAPQSSEFIGA- |
| SEQ ID NO: 56 | YLVIQGDER |
| SEQ ID NO: 57 | GSTAENAEYLR |
| SEQ ID NO: 58 | GSTAENAEY[Phosphoryl]LR |
| SEQ ID NO: 59 | GSHQISLDNPDYQQDDFFPK |
| SEQ ID NO: 60 | GSHQISLDNPDY[Phosphoryl] QQDDFFPK |
| SEQ ID NO: 61 | PAGSVQNPVYHNQPLNPAPSR |
| SEQ ID NO: 62 | PAGSVQNPVY[Phosphoryl]HNQPLNPAPSR |
| SEQ ID NO: 63 | ELVEPLTPSGEAPNQALLR |
| SEQ ID NO: 64 | ELVEPLTPS[Phosphoryl]GEAPNQALLR |
| SEQ ID NO: 65 | ELVEPLT[Phosphoryl]PSGEAPNQALLR |
| SEQ ID NO: 66 | ELVEPLT[Phosphoryl]PS[Phosphoryl]GEAPNQALLR |
| SEQ ID NO: 67 | GSHQISLDNPDYQQDFFPK |
| SEQ ID NO: 68 | GSHQISLDNPDY[Phosphoryl] QQDFFPK |
| SEQ ID NO: 69 | YSDPTGALTEDSIDDTFLPVPEYINQSVPK |
| SEQ ID NO: 70 | YSDPTGALTEDSIDDTFLPVPEY[Phosphoryl]INQSVPK |
| SEQ ID NO: 71 | Y[Phosphoryl]SDPTGALTEDSIDDTFLPVPEYINQSVPK |
| SEQ ID NO: 72 | Y[Phosphoryl]SDPTGALTEDSIDDTFLPVPEY[Phosphoryl]INQSVPK |
| SEQ ID NO: 73 | EYHAEGGK |
| SEQ ID NO: 74 | EY[Phosphoryl]HAEGGK |

| **Table 2 SEQ ID NO** | **Peptide sequence** | **Mono Isotopic Mass** | **Precursor Charge State** | **Precursor m/z** | **Transitio n m/z** | **Ion Type** |
|---|---|---|---|---|---|---|
| SEQ ID NO: 10 | GLWIPEGEK | 1027.534 | 2 | 514.774 | 559.272 | y5 |
| | | | 2 | 514.774 | 672.356 | y6 |
| | | | 2 | 514.774 | 858.435 | y7 |
| SEQ ID NO: 57 | GSTAENAEYLR | 1209.562 | 2 | 605.7880249 | 651.346 | y5 |
| | | | 2 | 605.7880249 | 765.388 | y6 |
| | | | 2 | 605.7880249 | 894.431 | y7 |
| SEQ ID NO: 22 | ITDFGLAK | 863.475 | 2 | 432.744 | 535.323 | y5 |
| | | | 2 | 432.744 | 650.350 | y6 |
| | | | 2 | 432.744 | 751.398 | y7 |
| SEQ ID NO: 39 | IPLENLQIIR | 1207.737 | 2 | 604.872 | 756.472 | y6 |
| | | | 2 | 604.872 | 885.515 | y7 |
| | | | 2 | 604.872 | 998.599 | y8 |
| SEQ ID NO: 58 | GSTAENAEY[Phosphoryl]LR | 1289.529 | 2 | 645.7709961 | 596.7833 | Reporter |
| | | | 2 | 645.7709961 | 660.2747 | y4 |
| | | | 2 | 645.7709961 | 731.3118 | y5 |
| | | | 2 | 645.7709961 | 845.3547 | y6 |
| | | | 2 | 645.7709961 | 974.3973 | y7 |
| | | | 2 | 645.7709961 | 1045.434 | y8 |
| | | | 2 | 645.7709961 | 1146.482 | y9 |
| | | | 2 | 645.7709961 | 1233.514 | y10 |
| | | | 2 | 645.7709961 | 1290.536 | y11 |
| SEQ ID NO: 64 | ELVEPLTPS[Phosphoryl]GEAPNQALLR | 2113.046 | 3 | 705.3549805 | 406.2426 | y7 |
| | | | 3 | 705.3549805 | 811.4779 | y7 |
| | | | 3 | 705.3549805 | 822.411 | y15 |
| SEQ ID NO: 65 | ELVEPLT [Phosphoryl]PS | 2113.046 | 2 | 1057.530029 | 1008.542 | Reporter |
| | GEAPNQALLR | | | | | |
| | | | 2 | 1057.530029 | 1252.664 | y12 |
| SEQ ID NO: 68 | GSHQISLDNPD | 2314.99 | 2 | 1158.501953 | 1364.555 | y10 |
| | Y[Phosphoryl]QQDFFPK | | | | | |
| | | | 2 | 1158.501953 | 1478.598 | y11 |

Protein lysates from cells procured directly from formalin (formaldehyde) fixed tissue were prepared using the Liquid Tissue™ reagents and protocol that entails collecting cells into a sample tube via tissue microdissection followed by heating the cells in the Liquid Tissue™ buffer for an extended period of time. Once the formalin-induced cross linking has been negatively affected, the tissue/cells are then digested to completion in a predictable manner using a protease, as for example including but not limited to the protease trypsin. Each protein lysate is turned into a collection of peptides by digestion of intact polypeptides with the protease. Each Liquid Tissue™ lysate was analyzed (e.g., by ion trap mass spectrometry) to perform multiple global proteomic surveys of the peptides where the data was presented as identification of as many peptides as could be identified by mass spectrometry from all cellular proteins present in each protein lysate. An ion trap mass spectrometer or another form of a mass spectrometer that is capable of performing global profiling for identification of as many peptides as possible from a single complex protein/peptide lysate is employed. Ion trap mass spectrometers however may be the best type of mass spectrometer for conducting global profiling of peptides. Although SRM/MRM assay can be developed and performed on any type of mass spectrometer, including a MALDI, ion trap, or triple quadrupole, the most advantageous instrument platform for SRM/MRM assay is often considered to be a triple quadrupole instrument platform.

Once as many peptides as possible were identified in a single MS analysis of a single lysate under the conditions employed, then that list of peptides was collated and used to determine the proteins that were detected in that lysate. That process was repeated for multiple Liquid Tissue™ lysates, and the very large list of peptides was collated into a single dataset. That type of dataset can be considered to represent the peptides that can be detected in the type of biological sample that was analyzed (after protease digestion), and specifically in a Liquid Tissue™ lysate of the biological sample, and thus includes the peptides for specific proteins, such as for example the EGFR protein.

In one embodiment of the invention as defined in the claims, the EGFR tryptic peptides identified as useful in the determination of absolute or relative amounts of the EGFR receptor include the peptide of SEQ ID NO: 4 and one or more, two or more, three or more, four or more, five or more, six or more, eight or more, or ten or more of the peptides of SEQ ID NO:1, SEQ ID NO:2, SEQ ID NO:3, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:38, SEQ ID NO:40, SEQ ID NO:41, SEQ ID NO:42, SEQ ID NO:43, SEQ ID NO:44, SEQ ID NO:45, SEQ ID NO:46, SEQ ID NO:47, SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62, SEQ ID NO:63, SEQ ID NO:64, SEQ ID NO:65, SEQ ID NO:66, SEQ ID NO:67, SEQ ID NO:68, SEQ ID NO:69, SEQ ID NO:70, SEQ ID NO:71, SEQ ID NO:72, SEQ ID NO:73, and SEQ ID NO:74, each of which are listed in Table 1. Each of those peptides, including the peptide of SEQ ID NO: 4, was detected by mass spectrometry in Liquid Tissue™ lysates prepared from formalin fixed, paraffin embedded tissue. Thus, each of the peptides in Table 1, or any combination of those peptides (e.g., one or more, two or more, three or more, four or more, five or more, six or more, eight or more, or ten or more of those peptides recited in Table 1, and particularly combinations with one or more of the peptides found in Table 2) are candidates for use in quantitative SRM/MRM assay for the EGRF protein in human biological samples, including directly in formalin fixed patient tissue.

The EGFR tryptic peptides listed in Table 1 include those detected from multiple Liquid Tissue™ lysates of multiple different formalin fixed tissues of different human organs including prostate, colon, and breast. Each of those peptides is considered useful for quantitative SRM/MRM assay of the EGFR protein in formalin fixed tissue. Further data analysis of these experiments indicated no preference is observed for any specific peptides from any specific organ site. Thus, each of these peptides is believed to be suitable for conducting SRM/MRM assays of the EGFR protein on a Liquid Tissue™ lysate from any formalin fixed tissue originating from any biological sample or from any organ site in the body.

It is disclosed that the peptides in Table 1, or any combination of those peptides (e.g., one or more, two or more, three or more, four or more, five or more, six or more, eight or more, or nine or more of those peptides recited in Table 1, and particularly combinations with the peptides also found in Table 2) are assayed by methods that do not rely upon mass spectroscopy, including, but not limited to, immunological methods (e.g., Western blotting or ELISA). Regardless of how information directed to the amount of the peptide(s) (absolute or relative) is obtained, the information may be employed in any of the methods described herein, including indicating (diagnosing) the presence of cancer in a subject, determining the stage/grade/status of the cancer, providing a prognosis, or determining the therapeutics or treatment regimen for a subject/patient.

The present disclosure also includes compositions comprising one or more, two or more, three or more, four or more, five or more, six or more, eight or more, or ten or more of the peptides in Table 1. The compositions may comprise one or more, two or more, three or more, four or more, five or more, six or more, or seven or more of the peptides in Table 2. Compositions comprising peptides may include one or more, two or more, three or more, four or more, five or more, six or more, eight or more, or ten or more peptides that are isotopically labeled. Each of the peptides may be labeled with one or more isotopes selected independently from the group consisting of: ¹⁸O, ¹⁷O, ³⁴S, ¹⁵N, ¹³C, ²H or combinations thereof. Compositions comprising peptides from the EGFR protein, whether isotope labeled or not, do not need to contain all of the peptides from that protein (e.g., a complete set of tryptic peptides). In some examples the compositions do not contain one or more, two or more, three or more, four or more, five or more, six or more, eight or more, or ten or more peptides from EGFR, and particularly peptides appearing in Table 1 or Table 2. Compositions comprising peptides may be in the form of dried or lyophilized materials, liquid (e.g., aqueous) solutions or suspensions, arrays, or blots.

An important consideration for conducting an SRM/MRM assay is the type of instrument that may be employed in the analysis of the peptides. Although SRM/MRM assays can be developed and performed on any type of mass spectrometer, including a MALDI, ion trap, or triple quadrupole, the most advantageous instrument platform for SRM/MRM assay is often considered to be a triple quadrupole instrument platform. That type of a mass spectrometer may be considered to be the most suitable instrument for analyzing a single isolated target peptide within a very complex protein lysate that may consist of hundreds of thousands to millions of individual peptides from all the proteins contained within a cell.

In order to most efficiently implement SRM/MRM assay for each peptide derived from the EGFR protein it is desirable to utilize information in addition to the peptide sequence in the analysis. That additional information may be used in directing and instructing the mass spectrometer (e.g. a triple quadrupole mass spectrometer), to perform the correct and focused analysis of specific targeted peptide(s), such that the assay may be effectively performed.

The additional information about target peptides in general, and about specific EGFR peptides, may include one or more of the mono isotopic mass of the peptide, its precursor charge state, the precursor m/z value, the m/z transition ions, and the ion type of each transition ion. Additional peptide information that may be used to develop an SRM/MRM assay for the EGFR protein is shown by example for eight (8) of the EGFR peptides from the list in Table 1 and is shown in Table 2. Similar additional information described for the eight (8) EGFR peptides shown by example in Table 2 may be prepared, obtained, and applied to the analysis of the other peptides contained in Table 1.

The method described below was used to: 1) identify candidate peptides from the EGFR protein that can be used for a mass spectrometry-based SRM/MRM assay for the EGFR protein, 2) develop individual SRM/MRM assay, or assays, for target peptides from the EGFR protein in order to correlate and 3) apply quantitative assays to cancer diagnosis and/or choice of optimal therapy.

### Assay Method

1. Identification of SRM/MRM candidate fragment peptides for the EGFR protein
   a. Prepare a Liquid Tissue™ protein lysate from a formalin fixed biological sample using a protease or proteases, (that may or may not include trypsin), to digest proteins
   b. Analyze all protein fragments in the Liquid Tissue™ lysate on an ion trap tandem mass spectrometer and identify all fragment peptides from the EGFR protein, where individual fragment peptides do not contain any peptide modifications such as phosphorylations or glycosylations
   c. Analyze all protein fragments in the Liquid Tissue™ lysate on an ion trap tandem mass spectrometer and identify all fragment peptides from the EGFR protein that carry peptide modifications such as for example phosphorylated or glycosylated residues
   d. All peptides generated by a specific digestion method from the entire, full length EGFR protein potentially can be measured, but preferred peptides used for development of the SRM/MRM assay are those that are identified by mass spectrometry directly in a complex Liquid Tissue™ protein lysate prepared from a formalin fixed biological sample
   e. Peptides that are specifically modified (phosphorylated, glycosylated, etc.) in patient tissue and which ionize, and thus detected, in a mass spectrometer when analyzing a Liquid Tissue™ lysate from a formalin fixed biological sample are identified as candidate peptides for assaying peptide modifications of the EGFR protein
2. Mass Spectrometry Assay for Fragment Peptides from EGFR Protein
   a. SRM/MRM assay on a triple quadrupole mass spectrometer for individual fragment peptides identified in a Liquid Tissue™ lysate is applied to peptides from the EGFR protein
      i. Determine optimal retention time for a fragment peptide for optimal chromatography conditions including but not limited to gel electrophoresis, liquid chromatography, capillary electrophoresis, nano-reversed phase liquid chromatography, high performance liquid chromatography, or reverse phase high performance liquid chromatography
      ii. Determine the mono isotopic mass of the peptide, the precursor charge state for each peptide, the precursor m/z value for each peptide, the m/z transition ions for each peptide, and the ion type of each transition ion for each fragment peptide in order to develop an SRM/MRM assay for each peptide.
      iii. SRM/MRM assay can then be conducted using the information from (i) and (ii) on a triple quadrupole mass spectrometer where each peptide has a characteristic and unique SRM/MRM signature peak that precisely defines the unique SRM/MRM assay as performed on a triple quadrupole mass spectrometer
   b. Perform SRM/MRM analysis so that the amount of the fragment peptide of the EGFR protein that is detected, as a function of the unique SRM/MRM signature peak area from an SRM/MRM mass spectrometry analysis, can indicate both the relative and absolute amount of the protein in a particular protein lysate.
      i. Relative quantitation may be achieved by:
         1. Determining increased or decreased presence of the EGFR protein by comparing the SRM/MRM signature peak area from a given EGFR peptide detected in a Liquid Tissue™ lysate from one formalin fixed biological sample to the same SRM/MRM signature peak area of the same EGFR fragment peptide in at least a second, third, fourth or more Liquid Tissue™ lysates from least a second, third, fourth or more formalin fixed biological samples
         2. Determining increased or decreased presence of the EGFR protein by comparing the SRM/MRM signature peak area from a given EGFR peptide detected in a Liquid Tissue™ lysate from one formalin fixed biological sample to SRM/MRM signature peak areas developed from fragment peptides from other proteins, in other samples derived from different and separate biological sources, where the SRM/MRM signature peak area comparison between the 2 samples for a peptide fragment are normalized to amount of protein analyzed in each sample.
         3. Determining increased or decreased presence of the EGFR protein by comparing the SRM/MRM signature peak area for a given EGFR peptide to the SRM/MRM signature peak areas from other fragment peptides derived from different proteins within the same Liquid Tissue™ lysate from the formalin fixed biological sample in order to normalize changing levels of EGFR protein to levels of other proteins that do not change their levels of expression under various cellular conditions.
         4. These assays can be applied to both unmodified fragment peptides and for modified fragment peptides of the EGFR protein, where the modifications include but are not limited to phosphorylation and/or glycosylation, and where the relative levels of modified peptides are determined in the same manner as determining relative amounts of unmodified peptides.
      ii. Absolute quantitation of a given peptide may be achieved by comparing the SRM/MRM signature peak area for a given fragment peptide from the EGFR protein in an individual biological sample to the SRM/MRM signature peak area of an internal fragment peptide standard spiked into the protein lysate from the biological sample
         1. The internal standard is a labeled synthetic version of the fragment peptide from the EGFR protein that is being interrogated. This standard is spiked into a sample in known amounts, and the SRM/MRM signature peak area can be determined for both the internal fragment peptide standard and the native fragment peptide in the biological sample separately, followed by comparison of both peak areas
         2. This can be applied to unmodified fragment peptides and modified fragment peptides, where the modifications include but are not limited to phosphorylation and/or glycosylation, and where the absolute levels of modified peptides can be determined in the same manner as determining absolute levels of unmodified peptides.
3. Apply Fragment Peptide Quantitation to Cancer Diagnosis and Treatment
   a. Perform relative and/or absolute quantitation of fragment peptide levels of the EGFR protein and demonstrate that the previously-determined association, as well understood in the field of cancer, of EGFR protein expression to the stage/grade/status of cancer in patient tumor tissue is confirmed
   b. Perform relative and/or absolute quantitation of fragment peptide levels of the EGFR protein and demonstrate correlation with clinical outcomes from different treatment strategies, wherein this correlation has already been demonstrated in the field or can be demonstrated in the future through correlation studies across cohorts of patients and tissue from those patients. Once either previously established correlations or correlations derived in the future are confirmed by this assay then the assay method can be used to determine optimal treatment strategy

Figure 1 shows an example of a single SRM/MRM assay performed on Liquid Tissue™ lysates from formalin fixed cancer tissue. An SRM/MRM assay was developed for a single peptide for quantitation of the EGFR protein on a triple quadrupole mass spectrometer. Specific and unique characteristics about this EGFR peptide (sequence IPLENLQIIR) were developed by analysis of all EGFR peptides on both an ion trap and triple quadrupole mass spectrometers and are shown in Figure 1A. That information includes the monoisotopic mass of the peptide, its precursor charge state, the precursor m/z value, the transition m/z values of the precursor, and the ion types of each of the identified transitions. That information must be determined experimentally for each and every candidate SRM/MRM peptide directly in Liquid Tissue™ lysates from formalin fixed tissue; because, interestingly, not all peptides from the EGFR protein can be detected in such lysates using SRM/MRM as described herein, indicating that EGFR peptides not detected cannot be considered candidate peptides for developing an SRM/MRM assay for use in quantitating peptides/proteins directly in Liquid Tissue™ lysates from formalin fixed tissue.

As shown in Figure 1B, this particular SRM/MRM assay was performed on a triple quadrupole mass spectrometer. A control protein lysate where the peptide was known to be present in large amounts was analyzed because this lysate was prepared from a mouse xenograft tumor that resulted from injection of a human-derived cancer cell line into a nude mouse. Thus, this xenograft tumor was the positive control. The experimental sample in this experiment was a Liquid Tissue™ protein lysate prepared from standard formalin fixed, paraffin embedded human breast cancer tissue. Data from the assay indicates the presence of the unique SRM/MRM signature peak for this EGFR peptide in both the control sample and the experimental sample. By comparing the SRM/MRM signature peak area between these 2 samples generates relative quantitative measure for the EGFR protein between 2 different biological samples.

Figure 1C shows quantitative measurement of the above-mentioned peptide across a collection of ten (10) formalin fixed cancer tissues using an internal standard to achieve absolute quantitation of the EGFR protein across a cohort of cancer-derived patient samples. These data indicate absolute amounts of this EGFR peptide as a function of molar amount of the peptide per microgram of protein lysate analyzed. Assessment of EGFR protein levels in tissues based on analysis of formalin fixed patient-derived tissue can provide diagnostic, prognostic, and therapeutically-relevant information about each particular patient. In a related embodiment of the invention as defined in the claims, quantifying one or more EGFR fragment peptides comprises determining the amount of the each of the EGFR fragment peptides in a biological sample by comparison to an added internal standard peptide of known amount, wherein each of the EGFR fragment peptides in the biological sample is compared to an internal standard peptide having the same amino acid sequence. In some embodiments of the invention as defined in the claims, the internal standard is an isotopically labeled internal standard peptide comprises one or more heavy stable isotopes selected from ¹⁸O, ¹⁷O, ³⁴S, ¹⁵N, ¹³C, ²H or combinations thereof.

The method of the invention as defined in the claims may be used as a diagnostic indicator of cancer in a patient or subject. In one embodiment, the results from measurements of the level of the EGFR protein may be employed to determine the diagnostic stage/grade/status of a cancer by correlating (e.g., comparing) the level of EGFR receptor found in a tissue with the level of that protein found in normal and/or cancerous or precancerous tissues.

## Claims

1. A method for measuring the level of the human Epidermal Growth Factor Receptor (EGFR) protein in a human biological sample of formalin fixed and paraffin embedded tissue, wherein the tissue is human breast cancer tissue, the method comprising detecting and quantifying the amount of a EGFR fragment peptide in a protein digest prepared from said human biological sample using mass spectrometry; and calculating the level of EGFR protein in said sample;
wherein the EGFR fragment peptide is the peptide of SEQ ID NO: 4, and
wherein said level is a relative level or an absolute level.

2. The method of claim 1, further comprising the step of fractionating said protein digest prior to detecting and quantifying the amount of said EGFR fragment peptide.

3. The method of any one of claims 1 to 2, wherein said protein digest comprises a protease digest.

4. The method of claim 1, wherein quantifying said EGFR fragment peptide comprises comparing the amount of said EGFR fragment peptide in one biological sample to the amount of the same EGFR fragment peptide in a different and separate biological sample.

5. The method of claim 1, wherein quantifying said EGFR fragment peptide comprises determining the amount of said EGFR fragment peptide in a biological sample by comparison to an added internal standard peptide of known amount, wherein said EGFR fragment peptide in the biological sample is compared to an internal standard peptide having the same amino acid sequence; and wherein the internal standard peptide is an isotopically labeled peptide.

6. The method of any one of claims 1 to 5, wherein detecting and quantifying the amount of said EGFR fragment peptide in the protein digest indicates the presence of modified or unmodified EGFR protein and an association with cancer in the subject.

7. The method of claim 6, further comprises correlating the results of said detecting and quantifying the amount of said EGFR fragment peptide, or the level of said EGFR protein to the diagnostic stage/grade/status of the cancer.

8. The method of claim 7, wherein correlating the results of said detecting and quantifying the amount of said EGFR fragment peptide, or the level of said EGFR protein to the diagnostic stage/grade/status of the cancer is combined with detecting and/or quantifying the amount of other proteins or peptides from other proteins in a multiplex format to provide additional information about the diagnostic stage/grade/status of the cancer.

## Patentansprüche

1. Verfahren zum Messen des Titers des menschlichen epidermalen Wachstumsfaktor-Rezeptor (EGFR)-Proteins in einer menschlichen biologischen Probe aus Formalin-fixiertem und in Paraffin eingebettetem Gewebe, wobei das Gewebe menschliches Brustkrebsgewebe ist, wobei das Verfahren umfasst Nachweisen und Quantifizieren der Menge eines EGFR-Fragmentpeptides in einem Proteinverdau, der von der menschlichen biologischen Probe hergestellt ist, unter Verwendung von Massenspektrometrie; und Berechnen des Titers von EGFR-Protein in der Probe;
wobei das EGFR-Fragmentpeptid das Peptid mit SEQ ID NO: 4 ist, und
wobei der Titer ein Relativtiter oder ein Absoluttiter ist.

2. Verfahren nach Anspruch 1, weiter umfassend den Schritt Fraktionieren des Proteinverdaus vor dem Nachweisen und Quantifizieren der Menge des EGFR-Fragmentpeptides.

3. Verfahren nach einem der Ansprüche 1 bis 2, wobei der Proteinverdau einen Proteaseverdau umfasst.

4. Verfahren nach Anspruch 1, wobei Quantifizieren des EGFR-Fragmentpeptides umfasst Vergleichen der Menge des EGFR-Fragmentpeptides in einer biologischen Probe mit der Menge desselben EGFR-Fragmentpeptides in einer verschiedenen und getrennten biologischen Probe.

5. Verfahren nach Anspruch 1, wobei Quantifizieren des EGFR-Fragmentpeptides umfasst Bestimmen der Menge des EGFR-Fragmentpeptides in einer biologischen Probe durch Vergleich mit einer bekannten Menge eines hinzugegebenen, als interner Standard dienenden Peptides, wobei das EGFR-Fragmentpeptid in der biologischen Probe verglichen wird mit einem als interner Standard dienenden Peptid mit derselben Aminosäuresequenz; und wobei das als interner Standard dienende Peptid ein Isotopen-markiertes Peptid ist.

6. Verfahren nach einem der Ansprüche 1 bis 5, wobei Nachweisen und Quantifizieren der Menge des EGFR-Fragmentpeptides in dem Proteinverdau die Anwesenheit von modifiziertem oder nicht modifiziertem EGFR-Protein und eine Verbindung mit Krebs in dem Lebewesen anzeigt.

7. Verfahren nach Anspruch 6, weiter umfassend Korrelieren der Ergebnisse des Nachweisens und Quantifizierens der Menge des EGFR-Fragmentpeptides oder des Titers des EGFR-Proteins mit dem diagnostischen Stadium/dem diagnostischen Grad/dem diagnostischen Status des Krebses.

8. Verfahren nach Anspruch 7, wobei Korrelieren der Ergebnisse des Nachweisens und Quantifizierens der Menge des EGFR-Fragmentpeptides oder des Titers des EGFR-Proteins mit dem diagnostischen Stadium/dem diagnostischen Grad/dem diagnostischen Status des Krebses kombiniert wird mit Nachweisen und/oder Quantifizieren der Menge von anderen Proteinen oder Peptiden von anderen Proteinen in einem Multiplex-Format, um zusätzliche Information über das diagnostische Stadium/den diagnostischen Grad/den diagnostischen Status des Krebses zu erhalten.

## Revendications

1. Procédé pour mesurer le taux de la protéine du récepteur du facteur de croissance de l'épiderme humain (EGFR) dans un échantillon biologique humain de tissu fixé au formol et enrobé de paraffine, dans lequel le tissu est un tissu de cancer mammaire humain, le procédé comprenant la détection et la quantification de la quantité d'un fragment peptidique d'EGFR dans une digestion de protéine préparée à partir dudit échantillon biologique humain en utilisant une spectrométrie de masse ; et le calcul du taux de la protéine d'EGFR dans ledit échantillon ;
dans lequel le fragment peptidique d'EGFR est le peptide de SEQ ID NO : 4, et
dans lequel ledit taux est un taux relatif ou un taux absolu.

2. Procédé selon la revendication 1, comprenant en outre l'étape de fractionnement de ladite digestion de protéine avant la détection et la quantification de la quantité dudit fragment peptidique d'EGFR.

3. Procédé selon l'une quelconque des revendications 1 à 2, dans lequel ladite digestion de protéine comprend une digestion par protéase.

4. Procédé selon la revendication 1, dans lequel la quantification dudit fragment peptidique d'EGFR comprend la comparaison de la quantité dudit fragment peptidique d'EGFR dans un échantillon biologique à la quantité du même fragment peptidique d'EGFR dans un échantillon biologique différent et séparé.

5. Procédé selon la revendication 1, dans lequel la quantification dudit fragment peptidique d'EGFR comprend la détermination de la quantité dudit fragment peptidique d'EGFR dans un échantillon biologique par comparaison avec un peptide étalon interne ajouté de quantité connue, dans lequel ledit fragment peptidique d'EGFR dans l'échantillon biologique est comparé à un peptide étalon interne ayant la même séquence d'acides aminés ; et dans lequel le peptide étalon interne est un peptide à marquage isotopique.

6. Procédé selon l'une quelconque des revendications 1 à 5, dans lequel la détection et la quantification de la quantité dudit fragment peptidique d'EGFR dans la digestion de protéine indique la présence d'une protéine d'EGFR modifiée ou non modifiée et une association avec un cancer chez le sujet.

7. Procédé selon la revendication 6, comprenant en outre la corrélation des résultats de ladite détection et de ladite quantification de la quantité dudit fragment peptidique d'EGFR, ou du taux de ladite protéine d'EGFR avec le stade/degré/état diagnostique du cancer.

8. Procédé selon la revendication 7, dans lequel la corrélation des résultats de ladite détection et de ladite quantification de la quantité dudit fragment peptidique d'EGFR, ou du taux de ladite protéine d'EGFR avec le stade/degré/état diagnostique du cancer est combinée avec la détection et/ou la quantification de la quantité d'autres protéines ou peptides provenant d'autres protéines dans un format multiplex pour fournir des informations supplémentaires concernant le stade/degré/état diagnostique du cancer.


### SEQUENCE LISTING

<110> Expression Pathology Inc. Krizman, David Thyparambil, Sheeno Hembrough, Todd
<120> Epidermal Growth Factor Receptor (EGFR) Protein SRM/MRM Assay
<130> 01152.8015
<150> US 61/289,384
   <151> 2009-12-22
<160> 74
<170> PatentIn version 3.5
<210> 1
   <211> 33
   <212> PRT
   <213> Artificial Sequence
<220>
   <223> EGFR peptide
<400> 1
   Cys Asp Pro Ser Cys Pro Asn Gly Ser Cys Trp Gly Ala Gly Glu Glu
   1               5                   10                  15
   Asn Cys Gln Lys Leu Thr Lys Ile Ile Cys Ala Gln Gln Cys Ser Gly
               20                  25                  30
   Arg
<210> 2
   <211> 7
   <212> PRT
   <213> Artificial Sequence
<220>
   <223> EGFR peptide
<400> 2
   Cys Glu Gly Pro Cys Arg Lys
   1               5
<210> 3
   <211> 7
   <212> PRT
   <213> Artificial Sequence
<220>
   <223> EGFR peptide
<400> 3
   Glu Asp Ser Phe Leu Gln Arg
   1 5
<210> 4
   <211> 10
   <212> PRT
   <213> Artificial Sequence
<220>
   <223> EGFR peptide
<400> 4
   Ile Pro Leu Glu Asn Leu Gln Ile Ile Arg
   1 5 10
<210> 5
   <211> 13
   <212> PRT
   <213> Artificial Sequence
<220>
   <223> EGFR peptide
<400> 5
   Glu Ile Ser Asp Gly Asp Val Ile Ile Ser Gly Asn Lys
   1 5 10
<210> 6
   <211> 15
   <212> PRT
   <213> Artificial Sequence
<220>
   <223> EGFR peptide
<400> 6
   Glu Ile Thr Gly Phe Leu Leu Ile Gln Ala Trp Pro Glu Asn Arg
   1 5 10 15
<210> 7
   <211> 12
   <212> PRT
   <213> Artificial Sequence
<220>
   <223> EGFR peptide
<400> 7
   Glu Leu Arg Glu Ala Thr Ser Pro Lys Ala Asn Lys
   1               5                   10
<210> 8
   <211> 8
   <212> PRT
   <213> Artificial Sequence
<220>
   <223> EGFR peptide
<400> 8
   Glu Tyr His Ala Glu Gly Gly Lys
   1               5
<210> 9
   <211> 10
   <212> PRT
   <213> Artificial Sequence
<220>
   <223> EGFR peptide
<400> 9
   Phe Arg Glu Leu Ile Ile Glu Phe Ser Lys
   1               5                   10
<210> 10
   <211> 9
   <212> PRT
   <213> Artificial Sequence
<220>
   <223> EGFR peptide
<400> 10
   Gly Leu Trp Ile Pro Glu Gly Glu Lys
   1               5
<210> 11
   <211> 17
   <212> PRT
   <213> Artificial Sequence
<220>
   <223> EGFR peptide
<400> 11
   Gly Leu Trp Ile Pro Glu Gly Glu Lys Val Lys Ile Pro Val Ala Ile
   1               5                   10                  15
   Lys
<210> 12
   <211> 39
   <212> PRT
   <213> Artificial Sequence
<220>
   <223> EGFR peptide
<400> 12
   His Phe Lys Asn Cys Thr Ser Ile Ser Gly Asp Leu His Ile Leu Pro
   1               5                   10                  15
   Val Ala Phe Arg Gly Asp Ser Phe Thr His Thr Pro Pro Leu Asp Pro
               20                  25                  30
   Gln Glu Leu Asp Ile Leu Lys
           35
<210> 13
   <211> 14
   <212> PRT
   <213> Artificial Sequence
<220>
   <223> EGFR peptide
<400> 13
   Ile Ile Cys Ala Gln Gln Cys Ser Gly Arg Cys Arg Gly Lys
   1               5                   10
<210> 14
   <211> 29
   <212> PRT
   <213> Artificial Sequence
<220>
   <223> EGFR peptide
<400> 14
   Ile Pro Ser Ile Ala Thr Gly Met Val Gly Ala Leu Leu Leu Leu Leu
   1               5                   10                  15
   Val Val Ala Leu Gly Ile Gly Leu Phe Met Arg Arg Arg
               20                  25
<210> 15
   <211> 6
   <212> PRT
   <213> Artificial Sequence
<220>
   <223> EGFR peptide
<400> 15
   Cys Glu Gly Pro Cys Arg
   1               5
<210> 16
   <211> 10
   <212> PRT
   <213> Artificial Sequence
<220>
   <223> EGFR peptide
<400> 16
   Leu Phe Gly Thr Ser Gly Gln Lys Thr Lys
   1               5                   10
<210> 17
   <211> 13
   <212> PRT
   <213> Artificial Sequence
<220>
   <223> EGFR peptide
<400> 17
   Leu Thr Lys Ile Ile Cys Ala Gln Gln Cys Ser Gly Arg
   1               5                   10
<210> 18
   <211> 36
   <212> PRT
   <213> Artificial Sequence
<220>
   <223> EGFR peptide
<400> 18
   Asn Cys Thr Ser Ile Ser Gly Asp Leu His Ile Leu Pro Val Ala Phe
   1 5 10 15
   Arg Gly Asp Ser Phe Thr His Thr Pro Pro Leu Asp Pro Gln Glu Leu
   20 25 30
   Asp Ile Leu Lys
   35
<210> 19
   <211> 19
   <212> PRT
   <213> Artificial Sequence
<220>
   <223> EGFR peptide
<400> 19
   Pro Tyr Asp Gly Ile Pro Ala Ser Glu Ile Ser Ser Ile Leu Glu Lys
   1 5 10 15
   Gly Glu Arg
<210> 20
   <211> 21
   <212> PRT
   <213> Artificial Sequence
<220>
   <223> EGFR peptide
<400> 20
   Pro Ala Gly Ser Val Gln Asn Pro Val Tyr His Asn Gln Pro Leu Asn
   1 5 10 15
   Pro Ala Pro Ser Arg
   20
<210> 21
   <211> 26
   <212> PRT
   <213> Artificial Sequence
<220>
   <223> EGFR peptide
<400> 21
   Ser Pro Ser Asp Cys Cys His Asn Gln Cys Ala Ala Gly Cys Thr Gly
   1 5 10 15
   Pro Arg Glu Ser Asp Cys Leu Val Cys Arg
   20 25
<210> 22
   <211> 8
   <212> PRT
   <213> Artificial Sequence
<220>
   <223> EGFR peptide
<400> 22
   Ile Thr Asp Phe Gly Leu Ala Lys
   1 5
<210> 23
   <211> 21
   <212> PRT
   <213> Artificial Sequence
<220>
   <223> EGFR peptide
<400> 23
   Thr Pro Gln His Val Lys Ile Thr Asp Phe Gly Leu Ala Lys Leu Leu
   1 5 10 15
   Gly Ala Glu Glu Lys
   20
<210> 24
   <211> 11
   <212> PRT
   <213> Artificial Sequence
<220>
   <223> EGFR peptide
<400> 24
   Val Cys Asn Gly Ile Gly Ile Gly Glu Phe Lys
   1 5 10
<210> 25
   <211> 25
   <212> PRT
   <213> Artificial Sequence
<220>
   <223> EGFR peptide
<400> 25
   Val Cys Asn Gly Ile Gly Ile Gly Glu Phe Lys Asp Ser Leu Ser Ile
   1               5                   10                  15
   Asn Ala Thr Asn Ile Lys His Phe Lys
               20                  25
<210> 26
   <211> 23
   <212> PRT
   <213> Artificial Sequence
<220>
   <223> EGFR peptide
<400> 26
   Val Leu Gly Ser Gly Ala Phe Gly Thr Val Tyr Lys Gly Leu Trp Ile
   1               5                   10                  15
   Pro Glu Gly Glu Lys Val Lys
               20
<210> 27
   <211> 13
   <212> PRT
   <213> Artificial Sequence
<220>
   <223> EGFR peptide
<400> 27
   Tyr Ser Phe Gly Ala Thr Cys Val Lys Lys Cys Pro Arg
   1               5                   10
<210> 28
   <211> 22
   <212> PRT
   <213> Artificial Sequence
<220>
   <223> EGFR peptide
<400> 28
   Cys Arg Gly Lys Ser Pro Ser Asp Cys Cys His Asn Gln Cys Ala Ala
   1               5                   10                  15
   Gly Cys Thr Gly Pro Arg
               20
<210> 29
   <211> 24
   <212> PRT
   <213> Artificial Sequence
<220>
   <223> EGFR peptide
<400> 29
   Asp Ile Val Ser Ser Asp Phe Leu Ser Asn Met Ser Met Asp Phe Gln
   1               5                   10                  15
   Asn His Leu Gly Ser Cys Gln Lys
               20
<210> 30
   <211> 27
   <212> PRT
   <213> Artificial Sequence
<220>
   <223> EGFR peptide
<400> 30
   Glu Phe Val Glu Asn Ser Glu Cys Ile Gln Cys His Pro Glu Cys Leu
   1               5                   10                  15
   Pro Gln Ala Met Asn Ile Thr Cys Thr Gly Arg
               20                  25
<210> 31
   <211> 15
   <212> PRT
   <213> Artificial Sequence
<220>
   <223> EGFR peptide
<400> 31
   Glu Leu Ile Ile Glu Phe Ser Lys Met Ala Arg Asp Pro Gln Arg
   1               5                   10                  15
<210> 32
   <211> 19
   <212> PRT
   <213> Artificial Sequence
<220>
   <223> EGFR peptide
<400> 32
   Glu Leu Val Glu Pro Leu Thr Pro Ser Gly Glu Ala Pro Asn Gln Ala
   1 5 10 15
   Leu Leu Arg
<210> 33
   <211> 11
   <212> PRT
   <213> Artificial Sequence
<220>
   <223> EGFR peptide
<400> 33
   Glu Ser Asp Cys Leu Val Cys Arg Lys Phe Arg
   1 5 10
<210> 34
   <211> 19
   <212> PRT
   <213> Artificial Sequence
<220>
   <223> EGFR peptide
<400> 34
   Gly Asp Ser Phe Thr His Thr Pro Pro Leu Asp Pro Gln Glu Leu Asp
   1 5 10 15
   Ile Leu Lys
<210> 35
   <211> 27
   <212> PRT
   <213> Artificial Sequence
<220>
   <223> EGFR peptide
<400> 35
   Gly Glu Asn Ser Cys Lys Ala Thr Gly Gln Val Cys His Ala Leu Cys
   1 5 10 15
   Ser Pro Glu Gly Cys Trp Gly Pro Glu Pro Arg
   20 25
<210> 36
   <211> 28
   <212> PRT
   <213> Artificial Sequence
<220>
   <223> EGFR peptide
<400> 36
   Gly Lys Ser Pro Ser Asp Cys Cys His Asn Gln Cys Ala Ala Gly Cys
   1               5                   10                  15
   Thr Gly Pro Arg Glu Ser Asp Cys Leu Val Cys Arg
               20                  25
<210> 37
   <211> 16
   <212> PRT
   <213> Artificial Sequence
<220>
   <223> EGFR peptide
<400> 37
   Gly Arg Glu Cys Val Asp Lys Cys Asn Leu Leu Glu Gly Glu Pro Arg
   1               5                   10                  15
<210> 38
   <211> 9
   <212> PRT
   <213> Artificial Sequence
<220>
   <223> EGFR peptide
<400> 38
   Ile Leu Lys Glu Thr Glu Phe Lys Lys
   1               5
<210> 39
   <211> 10
   <212> PRT
   <213> Artificial Sequence
<220>
   <223> EGFR peptide
<400> 39
   Ile Pro Leu Glu Asn Leu Gln Ile Ile Arg
   1               5                   10
<210> 40
   <211> 12
   <212> PRT
   <213> Artificial Sequence
<220>
   <223> EGFR peptide
<400> 40
   Lys Val Cys Asn Gly Ile Gly Ile Gly Glu Phe Lys
   1 5 10
<210> 41
   <211> 23
   <212> PRT
   <213> Artificial Sequence
<220>
   <223> EGFR peptide
<400> 41
   Lys Val Cys Asn Gly Ile Gly Ile Gly Glu Phe Lys Asp Ser Leu Ser
   1 5 10 15
   Ile Asn Ala Thr Asn Ile Lys
   20
<210> 42
   <211> 24
   <212> PRT
   <213> Artificial Sequence
<220>
   <223> EGFR peptide
<400> 42
   Leu Leu Gln Glu Arg Glu Leu Val Glu Pro Leu Thr Pro Ser Gly Glu
   1 5 10 15
   Ala Pro Asn Gln Ala Leu Leu Arg
   20
<210> 43
   <211> 13
   <212> PRT
   <213> Artificial Sequence
<220>
   <223> EGFR Peptide
<400> 43
   Met His Leu Pro Ser Pro Thr Asp Ser Asn Phe Tyr Arg
   1 5 10
<210> 44
   <211> 19
   <212> PRT
   <213> Artificial Sequence
<220>
   <223> EGFR Peptide
<400> 44
   Asn Val Leu Val Lys Thr Pro Gln His Val Lys Ile Thr Asp Phe Gly
   1               5                   10                  15
   Leu Ala Lys
<210> 45
   <211> 11
   <212> PRT
   <213> Artificial Sequence
<220>
   <223> EGFR Peptide
<400> 45
   Asn Val Ser Arg Gly Arg Glu Cys Val Asp Lys
   1               5                   10
<210> 46
   <211> 8
   <212> PRT
   <213> Artificial Sequence
<220>
   <223> EGFR Peptide
<400> 46
   Asn Tyr Asp Leu Ser Phe Leu Lys
   1               5
<210> 47
   <211> 12
   <212> PRT
   <213> Artificial Sequence
<220>
   <223> EGFR Peptide
<400> 47
   Pro Lys Phe Arg Glu Leu Ile Ile Glu Phe Ser Lys
   1               5                   10
<210> 48
   <211> 24
   <212> PRT
   <213> Artificial Sequence
<220>
   <223> EGFR Peptide
<400> 48
   Leu Leu Gln Glu Arg Glu Leu Val Glu Pro Leu Thr Pro Ser Gly Glu
   1 5 10 15
   Ala Pro Asn Gln Ala Leu Leu Arg
   20
<210> 49
   <211> 16
   <212> PRT
   <213> Artificial Sequence
<220>
   <223> EGFR Peptide
<400> 49
   Ser Leu Lys Glu Ile Ser Asp Gly Asp Val Ile Ile Ser Gly Asn Lys
   1 5 10 15
<210> 50
   <211> 13
   <212> PRT
   <213> Artificial Sequence
<220>
   <223> EGFR Peptide
<400> 50
   Thr Asp Leu His Ala Phe Glu Asn Leu Glu Ile Ile Arg
   1 5 10
<210> 51
   <211> 15
   <212> PRT
   <213> Artificial Sequence
<220>
   <223> EGFR Peptide
<400> 51
   Thr Asp Leu His Ala Phe Glu Asn Leu Glu Ile Ile Arg Gly Arg
   1 5 10 15
<210> 52
   <211> 22
   <212> PRT
   <213> Artificial Sequence
<220>
   <223> EGFR Peptide
<400> 52
   Thr Lys Gln His Gly Gln Phe Ser Leu Ala Val Val Ser Leu Asn Ile
   1 5 10 15
   Thr Ser Leu Gly Leu Arg
   20
<210> 53
   <211> 9
   <212> PRT
   <213> Artificial Sequence
<220>
   <223> EGFR Peptide
<400> 53
   Thr Leu Arg Arg Leu Leu Gln Glu Arg
   1 5
<210> 54
   <211> 21
   <212> PRT
   <213> Artificial Sequence
<220>
   <223> EGFR Peptide
<400> 54
   Thr Pro Leu Leu Ser Ser Leu Ser Ala Thr Ser Asn Asn Ser Thr Val
   1 5 10 15
   Ala Cys Ile Asp Arg
   20
<210> 55
   <211> 11
   <212> PRT
   <213> Artificial Sequence
<220>
   <223> EGFR Peptide
<400> 55
   Val Ala Pro Gln Ser Ser Glu Phe Ile Gly Ala
   1 5 10
<210> 56
   <211> 9
   <212> PRT
   <213> Artificial Sequence
<220>
   <223> EGFR Peptide
<400> 56
   Tyr Leu Val Ile Gln Gly Asp Glu Arg
   1 5
<210> 57
   <211> 11
   <212> PRT
   <213> Artificial Sequence
<220>
   <223> EGFR Peptide
<400> 57
   Gly Ser Thr Ala Glu Asn Ala Glu Tyr Leu Arg
   1               5                   10
<210> 58
   <211> 11
   <212> PRT
   <213> Artificial Sequence
<220>
   <223> EGFR Peptide
<220>
   <221> MOD_RES
   <222> (9) .. (9)
   <223> PHOSPHORYLATION
<400> 58
   Gly Ser Thr Ala Glu Asn Ala Glu Tyr Leu Arg
   1               5                   10
<210> 59
   <211> 20
   <212> PRT
   <213> Artificial Sequence
<220>
   <223> EGRF Peptide
<400> 59
   Gly Ser His Gln Ile Ser Leu Asp Asn Pro Asp Tyr Gln Gln Asp Asp
   1               5                   10                  15
   Phe Phe Pro Lys
               20
<210> 60
   <211> 20
   <212> PRT
   <213> Artificial Sequence
<220>
   <223> EGRF Peptide
<220>
   <221> MOD_RES
   <222> (12)..(12)
   <223> PHOSPHORYLATION
<400> 60
   Gly Ser His Gln Ile Ser Leu Asp Asn Pro Asp Tyr Gln Gln Asp Asp
   1               5                   10                  15
   Phe Phe Pro Lys
               20
<210> 61
   <211> 21
   <212> PRT
   <213> Artificial Sequence
<220>
   <223> EGRF Peptide
<400> 61
   Pro Ala Gly Ser Val Gln Asn Pro Val Tyr His Asn Gln Pro Leu Asn
   1               5                   10                  15
   Pro Ala Pro Ser Arg
               20
<210> 62
   <211> 21
   <212> PRT
   <213> Artificial Sequence
<220>
   <223> EGRF Peptide
<220>
   <221> MOD_RES
   <222> (10)..(10)
   <223> PHOSPHORYLATION
<400> 62
   Pro Ala Gly Ser Val Gln Asn Pro Val Tyr His Asn Gln Pro Leu Asn
   1               5                   10                  15
   Pro Ala Pro Ser Arg
               20
<210> 63
   <211> 19
   <212> PRT
   <213> Artificial Sequence
<220>
   <223> EGRF Peptide
<400> 63
   Glu Leu Val Glu Pro Leu Thr Pro Ser Gly Glu Ala Pro Asn Gln Ala
   1 5 10 15
   Leu Leu Arg
<210> 64
   <211> 19
   <212> PRT
   <213> Artificial Sequence
<220>
   <223> EGRF Peptide
<220>
   <221> MOD_RES
   <222> (9) .. (9)
   <223> PHOSPHORYLATION
<400> 64
   Glu Leu Val Glu Pro Leu Thr Pro Ser Gly Glu Ala Pro Asn Gln Ala
   1 5 10 15
   Leu Leu Arg
<210> 65
   <211> 19
   <212> PRT
   <213> Artificial Sequence
<220>
   <223> EGRF Peptide
<220>
   <221> MOD_RES
   <222> (7)..(7)
   <223> PHOSPHORYLATION
<400> 65
   Glu Leu Val Glu Pro Leu Thr Pro Ser Gly Glu Ala Pro Asn Gln Ala
   1 5 10 15
   Leu Leu Arg
<210> 66
   <211> 19
   <212> PRT
   <213> Artificial Sequence
<220>
   <223> EGRF Peptide
<220>
   <221> MOD_RES
   <222> (7)..(7)
   <223> PHOSPHORYLATION
<220>
   <221> MOD_RES
   <222> (9) .. (9)
   <223> PHOSPHORYLATION
<400> 66
   Glu Leu Val Glu Pro Leu Thr Pro Ser Gly Glu Ala Pro Asn Gln Ala
   1 5 10 15
   Leu Leu Arg
<210> 67
   <211> 19
   <212> PRT
   <213> Artificial Sequence
<220>
   <223> EGRF Peptide
<400> 67
   Gly Ser His Gln Ile Ser Leu Asp Asn Pro Asp Tyr Gln Gln Asp Phe
   1 5 10 15
   Phe Pro Lys
<210> 68
   <211> 19
   <212> PRT
   <213> Artificial Sequence
<220>
   <223> EGRF Peptide
<220>
   <221> MOD_RES
   <222> (12)..(12)
   <223> PHOSPHORYLATION
<400> 68
   Gly Ser His Gln Ile Ser Leu Asp Asn Pro Asp Tyr Gln Gln Asp Phe
   1 5 10 15
   Phe Pro Lys
<210> 69
   <211> 30
   <212> PRT
   <213> Artificial Sequence
<220>
   <223> EGRF Peptide
<400> 69
   Tyr Ser Asp Pro Thr Gly Ala Leu Thr Glu Asp Ser Ile Asp Asp Thr
   1 5 10 15
   Phe Leu Pro Val Pro Glu Tyr Ile Asn Gln Ser Val Pro Lys
   20 25 30
<210> 70
   <211> 30
   <212> PRT
   <213> Artificial Sequence
<220>
   <223> EGRF Peptide
<220>
   <221> MOD_RES
   <222> (23) .. (23)
   <223> PHOSPHORYLATION
<400> 70
   Tyr Ser Asp Pro Thr Gly Ala Leu Thr Glu Asp Ser Ile Asp Asp Thr
   1 5 10 15
   Phe Leu Pro Val Pro Glu Tyr Ile Asn Gln Ser Val Pro Lys
   20 25 30
<210> 71
   <211> 30
   <212> PRT
   <213> Artificial Sequence
<220>
   <223> EGRF Peptide
<220>
   <221> MOD_RES
   <222> (1) .. (1)
   <223> PHOSPHORYLATION
<400> 71
   Tyr Ser Asp Pro Thr Gly Ala Leu Thr Glu Asp Ser Ile Asp Asp Thr
   1 5 10 15
   Phe Leu Pro Val Pro Glu Tyr Ile Asn Gln Ser Val Pro Lys
   20 25 30
<210> 72
   <211> 30
   <212> PRT
   <213> Artificial Sequence
<220>
   <223> EGRF Peptide
<220>
   <221> MOD_RES
   <222> (1) .. (1)
   <223> PHOSPHORYLATION
<220>
   <221> MOD_RES
   <222> (23) .. (23)
   <223> PHOSPHORYLATION
<400> 72
   Tyr Ser Asp Pro Thr Gly Ala Leu Thr Glu Asp Ser Ile Asp Asp Thr
   1               5                   10                  15
   Phe Leu Pro Val Pro Glu Tyr Ile Asn Gln Ser Val Pro Lys
               20                  25                  30
<210> 73
   <211> 8
   <212> PRT
   <213> Artificial Sequence
<220>
   <223> EGRF Peptide
<400> 73
   Glu Tyr His Ala Glu Gly Gly Lys
   1               5
<210> 74
   <211> 8
   <212> PRT
   <213> Artificial Sequence
<220>
   <223> EGRF Peptide
<220>
   <221> MOD_RES
   <222> (2) .. (2)
   <223> PHOSPHORYLATION
<400> 74
   Glu Tyr His Ala Glu Gly Gly Lys
   1               5